(19) Europäisches Patentamt
European Patent Office
Office européen des brevets

(11) **EP 3 839 503 A1**

(12) **DEMANDE DE BREVET EUROPEEN**

(43) Date de publication:
**23.06.2021 Bulletin 2021/25**

(51) Int Cl.:
***G01N 33/28*** *(2006.01)*   ***G01N 11/00*** *(2006.01)*

(21) Numéro de dépôt: **19306674.3**

(22) Date de dépôt: **18.12.2019**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
**KH MA MD TN**

(71) Demandeur: **Total Raffinage Chimie**
**92400 Courbevoie (FR)**

(72) Inventeurs:
• **HEYBERGER, Barbara**
  **27520 THENOUVILLE (FR)**
• **LE ROUX, Anthony**
  **76430 SAINT AUBIN ROUTOT (FR)**

(74) Mandataire: **Blaise, Lucie**
**Total Recherche & Technologie Feluy**
**Patent Department**
**Zone Industrielle C**
**7181 Seneffe (BE)**

(54) **MÉTHODE D'ESTIMATION DU POUVOIR SOLVANT SO D'UN FLUXANT ET MÉTHODE DE PRÉDICTION DE LA STABILITÉ D'UN MÉLANGE DE FLUX HYDROCARBONÉ(S) CONTENANT DES ASPHALTÈNES ET AU MOINS UN FLUXANT**

(57)    L'invention concerne une méthode d'estimation du pouvoir solvant So d'un fluxant, notamment d'un fluxant dont le pouvoir solvant So n'est pas mesurable selon la norme ASTM D7157, notamment selon la norme ASTM D7157-18 (Révision 2018), à partir de caractéristiques physiques du fluxant. L'invention concerne également une méthode de prédiction de la stabilité d'un mélange comprenant au moins un flux hydrocarboné contenant des asphaltènes et au moins un tel fluxant.

**EP 3 839 503 A1**

**Description**

Domaine de l'invention

L'invention concerne une méthode d'estimation du pouvoir solvant So d'un fluxant, notamment d'un fluxant dont le pouvoir solvant So n'est pas mesurable selon la norme ASTM D7157, notamment la norme ASTMD1757-18 (Révision 2018). L'invention concerne également une méthode de prédiction de la stabilité d'un mélange comprenant au moins un flux hydrocarboné contenant des asphaltènes et au moins un fluxant.

Art antérieur

Les produits pétroliers, et notamment les combustibles lourds tels que les fiouls ou les résidus de distillation du pétrole, généralement appelés "Produits noirs" dans la profession, sont des systèmes colloïdaux comprenant des asphaltènes - c'est à dire de molécules lourdes très aromatiques possédant des chaînes latérales paraffiniques - qui sont dispersés (ou "peptisés") sous forme de micelles dans une phase huileuse. Ces systèmes colloïdaux peuvent être déstabilisés plus ou moins facilement, par exemple par craquage ou dilution. Les asphaltènes floculent alors en s'agglomérant sous forme de grosses particules pouvant générer des colmatages, encrassement et autres problèmes bien connus. Notamment, la constitution de mélanges contenant de tels systèmes colloïdaux peut générer une précipitation de ces asphaltènes par floculation, en particulier si l'environnement de dilution est du type paraffinique.

On notera que les fiouls ou résidus de pétrole sont constitués d'une matrice malténique (résines + paraffines) et d'asphaltènes dispersés sous forme colloïdale. Les asphaltènes qui ont un caractère très aromatique sont insolubles dans les paraffines qui ont un caractère aliphatique. Pour qu'un résidu soit stable, il est nécessaire que les asphaltènes soient maintenus en suspension (ou dispersés ou peptisés) dans la matrice huileuse. La peptisation des asphaltènes est assurée par les résines qui ont à la fois un caractère aromatique et un caractère aliphatique.

Pour prédire la stabilité d'un produit noir, on utilise généralement la caractéristique appelée S-value, ou encore stabilité intrinsèque, qui est définie dans la profession ainsi que dans la norme ASTM D7157-18 (Révision 2018) par l'expression suivante :

$$S = \text{aromaticité des maltènes/aromaticité des asphaltènes,}$$

soit encore S=So/(1-Sa), dans laquelle :

- So représente le pouvoir du milieu à solubiliser les asphaltènes (pouvoir solvant), c'est à dire le caractère aromatique du milieu. Plus celui-ci sera aromatique, plus le So sera élevé,
- Sa représente la peptisabilité des asphaltènes,
- 1-Sa représente l'aromaticité du milieu nécessaire pour solubiliser les asphaltènes présents.

On peut par exemple considérer les domaines suivants en termes de stabilité :

- S-Value $\geq$ 1,50 : mélange stable
- S-Value < 1,35 : mélange instable
- 1,35 $\leq$ S-Value < 1,50 : zone critique

Les paramètres de S-value répondent à une loi de mélange qui peut ainsi être utilisée pour prédire la stabilité d'un mélange de constituants (i.e. la compatibilité des constituants de ce mélange dans des proportions définies) à condition de connaître les valeurs du pouvoir solvant So et la peptisabilité des asphaltènes Sa des constituants et de fixer une cible de S-value appropriée. Les lois de mélange de ces paramètres s'écrivent :

$$So_{mélange} = \sum x_i \times So_i \qquad \text{(équation 1)}$$

$$Sa_{mélange} = \frac{\sum x_i \times ASP_i \times Sa_i}{\sum x_i \times ASP_i} \qquad \text{(équation 2)}$$

$$S_{mélange} = \frac{So_{mélange}}{1-Sa_{mélange}} \qquad \text{(équation 3)}$$

Où :

$x_i$ est la fraction massique du composant i,
$ASP_i$ est la teneur en asphaltènes (%m) du composant i,
$So_i$ est le pouvoir solvant du composant i,
$Sa_i$ est la peptisabilité du composant i.

On notera que l'équation (1) est une forme simplifiée de l'équation (1bis) ci-dessous

$$So_{mélange} = \frac{\sum x_i \times (100 - ASP_i) \times So_i}{\sum x_i \times (100 - ASP_i)} \qquad \text{(équation 1bis)}.$$

C'est ainsi que, si on ajoute un fluxant de pouvoir solvant faible à un produit noir, par exemple viscoréduit, qui présente des valeurs So élevée et Sa faible, on réduit la valeur du So du mélange, ce qui peut conduire à une déstabilisation du produit noir, et par conséquent à une floculation des asphaltènes, car les valeurs So et Sa résultantes seraient trop faibles pour satisfaire la relation S≥1,5, c'est à dire la condition pour que les asphaltènes soient peptisés, donc stables.
Aujourd'hui, les fluxants contenus dans les compositions de combustibles lourds sont des fluxants aromatiques contenant ou non des asphaltènes dont le pouvoir solvant So est suffisant pour assurer la peptisation des asphaltènes et donc la stabilité du mélange. Avec l'entrée en vigueur prochaine de la nouvelle règlementation soufre des combustibles pour la marine, ces fluxants aromatiques pourront être remplacés par des fluxants paraffiniques, notamment exempts d'asphaltènes. L'utilisation de résidus moins lourds est également envisagée.
Or, la mesure de la S-value et des paramètres de stabilité d'hydrocarbures selon la norme ASTM D7157-18 (Révision 2018) (ou les plus anciennes versions de la norme) n'est pas toujours possible en fonction de la nature des asphaltènes présents et/ou lorsque la teneur en asphaltènes est insuffisante.
Il existe donc un besoin pour déterminer le pouvoir solvant So de fluxant(s) qui peuvent entrer dans les formulations de combustibles lourds, notamment pour la marine, lorsque le pouvoir solvant So de ce(s) fluxant(s) n'est pas mesurable selon la norme ASTM D7157, notamment selon la norme ASTM D7157-18 (Révision 2018) ou les autres versions de la norme.
La méthode selon la présente invention permet de déterminer le pouvoir solvant So d'un tel fluxant à partir de propriétés physiques du fluxant, telles que les viscosités cinématiques à 50 et 100°C et la masse volumique à 15°C.
Dès lors, à partir du So du ou des fluxants et des paramètres Sa et So des autres composants contenant des asphaltènes, tels que les résidus, on peut déterminer la valeur de la S-value et prédire la stabilité d'un mélange. Notamment, on notera que la loi de mélange du paramètre Sa prend uniquement en compte la contribution des composants contenant des asphaltènes alors que la loi de mélange du paramètre So (simplifiée ou non) prend en compte la contribution du pouvoir solvant de tous les composants, quelle que soit leur teneur en asphaltènes.

Définitions

Par « flux hydrocarboné », on entend un mélange de composés hydrocarbonés, un composé hydrocarboné contenant du carbone et de l'hydrogène, et éventuellement un ou plusieurs hétéroatomes tels que le soufre, l'azote, des métaux, diverses impuretés .... Les flux hydrocarbonés considérés ici sont sous forme liquide à une température donnée.
Un mélange de flux hydrocarbonés contenant des asphaltènes est un mélange d'au moins deux flux hydrocarbonés, dont au moins un contient des asphaltènes.
Par « asphaltènes », on entend les composés insolubles dans le n-heptane et solubles dans le toluène contenus notamment dans les pétroles bruts, les bitumes, le charbon. En général, les asphaltènes comprennent du carbone, de l'hydrogène, de l'azote, du soufre, du vanadium et du nickel.
Par « paramètre de stabilité », on entend un paramètre permettant de déterminer la stabilité d'un flux hydrocarboné ou de prédire la stabilité d'un mélange de flux hydrocarbonés, c'est-à-dire la compatibilité de ces flux hydrocarbonés entre eux et dans certaines proportions.
Un mélange de constituants, notamment de flux hydrocarbonés, est stable (ou des flux hydrocarbonés sont compatibles), si aucune précipitation d'asphaltènes n'est observée. La précipitation d'asphaltènes peut être observée et constatée par un observateur, par l'observation de la formation d'un dépôt au fond d'une cuve de stockage ou tout autre moyen approprié.

Habituellement, on entend par mélange stable, un mélange stable au sens de la norme ISO 8217 :2017, c'est-à-dire dont la teneur en sédiments potentiels mesurée selon la norme ISO 10307-2A :2009 est inférieure ou égale à 0,10% masse.

« Fluxant » désigne un flux hydrocarboné qui permet de modifier une ou plusieurs caractéristiques physiques d'un autre flux hydrocarboné afin qu'elles soient conformes à des spécifications. La ou les caractéristiques physiques susceptibles d'être modifiées par l'ajout d'un fluxant sont choisies parmi : la viscosité, la masse volumique, la teneur en soufre, le point d'écoulement ou encore le résidu de carbone.

« Pétrole brut » (ou « brut », *crude oil* en anglais) désigne le pétrole issu d'un gisement naturel, et que l'on exploite sous forme liquide à la pression atmosphérique. Cette appellation désigne donc un produit naturel avant raffinage, mais qui a déjà perdu une partie de sa composition de gisement, la fraction d'hydrocarbures légers quittant la phase liquide sur le lieu même de son exploitation.

« Produit pétrolier » désigne un effluent d'une unité de traitement d'un pétrole brut ou d'un effluent, ou un effluent d'une unité de séparation d'un pétrole brut ou d'un effluent, ou encore des produits pétroliers non valorisables tels que les slops. Les produits pétroliers lourds sont des mélanges de température d'ébullition supérieure ou égale à 350°C, noté 350°C+. Il s'agit notamment des résidus de distillation du pétrole, des effluents issus de procédés de conversion thermique, de procédés de craquage catalytique, de procédés d'hydrocraquage, de procédés d'hydroconversion profonde, de procédés d'hydrotraitement des résidus atmosphériques ou sous vide (ARDS ou VRDS), ou bien encore les fiouls issus de mélanges de produits lourds.

Les produits pétroliers contenant des asphaltènes peuvent être :

- des résidus atmosphériques ou des résidus sous vide issus de la distillation du pétrole brut qui contiennent classiquement de 0,01% à 25% m d'asphaltènes.
- des effluents, notamment des résidus, issus des procédés de conversion thermique tel que le procédé de viscoréduction, qui contiennent classiquement de 5 à 30% m d'asphaltènes.
- des effluents, notamment des résidus, issus de procédés de craquage catalytique, tel que le procédé FCC (« Fluid Catalytic Cracking » ou craquage catalytique fluide), et dont la coupe slurry (coupe 350°C+) contient classiquement de 0,1 à 8% m d'asphaltènes.
- des effluents, notamment des résidus, issus des procédés d'hydrotraitement, d'hydrocraquage, d'hydroconversion profonde (à lit fixe, à lit mobile, lit bouillonnant, lit entraîné, ou en réacteur en phase slurry (dont le catalyseur est en suspension)), ou du procédé ARDS (« Atmospheric Residue DeSulfurization » ou Désulfuration de résidu atmosphérique) ou VRDS (« Vacuum Residu DeSulfurization » ou Désulfuration de résidu sous vide) et qui contiennent classiquement jusqu'à 50%m d'asphaltènes.
- des brais issus de procédés de séparation physique, tel que le désasphaltage, qui contiennent classiquement de 4 à 50%m d'asphaltènes.
- des mélanges des produits pétroliers listés ci-dessus pour la formulation de fuels lourds qui contiennent classiquement de 0,20 à 20%m d'asphaltènes.

Résumé de l'invention

L'invention concerne une méthode d'estimation de la valeur du pouvoir solvant So d'un fluxant pour composition combustible. La présente invention est plus particulièrement adaptée pour estimer la valeur du pouvoir solvant So d'un fluxant lorsque ce pouvoir solvant n'est pas mesurable par la norme ASTM D7157, notamment selon la norme ASTM D7157-18 (Révision 2018) ou d'autres versions de la norme.

Le pouvoir solvant So, la peptisabilité des asphaltènes Sa et la S-value sont ici tels que définis dans la norme ASTM D7157-18 (Révision 2018).

La méthode selon l'invention comprend :

(a) établir une corrélation exprimant le pouvoir solvant So d'un fluxant ou d'un mélange de fluxants en fonction de la viscosité cinématique à 50°C, la viscosité cinématique à 100°C et la masse volumique à 15°C dudit fluxant ou mélange de fluxants,
(b) utiliser ladite corrélation pour estimer la valeur du pouvoir solvant So d'un fluxant ou d'un mélange de fluxants dont on a mesuré la viscosité cinématique à 50°C, la viscosité cinématique à 100°C et la masse volumique à 15°C,

l'étape (a) d'établissement de la corrélation comprenant :

(i) mesurer la viscosité cinématique à 50°C, la viscosité cinématique à 100°C et la masse volumique à 15°C d'une pluralité de fluxants et/ou de mélanges de fluxants,

(ii) prévoir des flux hydrocarbonés dont il est possible de mesurer une valeur de pouvoir solvant So selon la norme ASTM D7157, notamment la norme ASTM D7157-18 (Révision 2018) ou une autre version de la norme,

(iii) préparer au moins deux mélanges de différentes proportions de chacun des flux hydrocarbonés avec chacun des fluxants et mélanges de fluxants,

(iv) pour chacun des mélanges comprenant un même fluxant ou mélange de fluxants, mesurer la valeur du pouvoir solvant So, notamment selon la norme ASTM D7157, en particulier selon la norme ASTM D7157-18 (Révision 2018) ou une autre version de la norme, et tracer, pour chaque flux hydrocarboné, une droite représentant cette valeur du pouvoir solvant So en fonction de la proportion de fluxant ou de mélange de fluxants dans le mélange et, pour chaque droite tracée, déterminer une valeur du pouvoir solvant So du fluxant ou du mélange de fluxants par extrapolation à une proportion de fluxant de 100%, puis calculer une valeur moyenne de ces valeurs de pouvoir solvant So pour chaque fluxant et mélange de fluxants,

(v) établir ladite corrélation par un traitement statistique des valeurs moyennes de pouvoir solvant So, des valeurs de viscosité cinématique à 50°C, de viscosité cinématique à 100°C et de masse volumique à 15°C de tous les fluxants et mélanges de fluxants.

L'établissement de la corrélation permet ainsi d'estimer le pouvoir solvant So d'un fluxant à partir de données physiques qui sont généralement connues (viscosités cinématiques à 50° et 100°C, masse volumique à 15°C). Il est dès lors possible de prédire la stabilité d'un mélange de fluxants et de flux hydrocarbonés à partir de la valeur So prédite du ou des fluxants et des paramètres Sa et So du ou des flux hydrocarbonés à mélanger au(x) fluxant(s).

Par exemple, les mesures des viscosités cinématiques à 50°C et à 100°C peuvent être réalisées selon la norme NF EN ISO 3104 (janvier 2018), et la mesure de la masse volumique à 15°C peut être réalisée selon la norme NF EN ISO 12185 (décembre 1996). D'autres normes peuvent néanmoins être utilisées pourvu que les mêmes normes soient utilisées pour établir la corrélation et pour l'étape (b).

L'invention concerne également une méthode de prédiction de la stabilité d'un mélange comprenant au moins un flux hydrocarboné contenant des asphaltènes et au moins un fluxant, comprenant :

- estimer la valeur du pouvoir solvant So dudit au moins un fluxant en mettant en œuvre la méthode d'estimation selon l'invention,
- mesurer la valeur du pouvoir solvant So et la valeur de la peptisabilité des asphaltènes Sa dudit au moins un flux hydrocarboné selon la norme ASTM D7157, notamment selon la norme ASTM D7157-18 (Révision 2018) ou selon une autre version de la norme,
- calculer la valeur du pouvoir solvant $So_{mélange}$ du mélange et la valeur de la peptisabilité des asphaltènes $Sa_{mélange}$ du mélange en utilisant les lois de mélange suivantes :

$$So_{mélange} = \sum x_i \times So_i \qquad \text{(équation 1)}$$

ou

$$\text{ou } So_{mélange} = \frac{\sum x_i \times (100 - ASP_i) \times So_i}{\sum x_i \times (100 - ASP_i)} \qquad \text{(équation 1bis),}$$

et

$$Sa_{mélange} = \frac{\sum x_i \times ASP_i \times Sa_i}{\sum x_i \times ASP_i} \qquad \text{(équation 2)}$$

- calculer la valeur de la S-value du mélange $Svalue_{mélange}$ définie comme étant égale à $S_{mélange} = \frac{So_{mélange}}{1 - Sa_{mélange}}$,
- prédire que :

le mélange est stable si la valeur $S_{mélange}$ est supérieure ou égale à une valeur cible,
le mélange est instable si la valeur $S_{mélange}$ est inférieure à la même valeur cible.

Cette valeur cible est égale à la valeur 1,5 augmentée ou réduite d'une quantité prédéterminée nulle ou non nulle.

La présente invention permet ainsi de déterminer la stabilité d'un mélange sans faire appel à la norme ISO 8217 :2017, c'est-à-dire sans avoir à déterminer la teneur en sédiments potentiels mesurée selon la norme ISO 10307-2A : 2009.

Toutefois, lorsque la valeur $S_{mélange}$ est inférieure à la valeur cible, augmentée ou réduite d'une quantité prédéterminée nulle ou non nulle, tout en étant proche de celle-ci, le mélange peut quand même être stable. Cette stabilité peut alors être confirmée par une autre méthode.

Ainsi, dans un mode de réalisation, on peut prédire que le mélange est instable si la valeur $S_{mélange}$ est inférieure à une valeur seuil égale à la valeur cible réduite d'une quantité correspondant à une marge d'erreur. Cette marge d'erreur, non nulle, peut représenter 1 à 50% de la valeur cible.

Dans ce cas, lorsque la valeur $S_{mélange}$ est supérieure ou égale à la valeur seuil et inférieure à la valeur cible, on vérifie que le mélange est stable si sa teneur en sédiments potentiels mesurée selon la norme ISO 10307-2A :2009 est inférieure ou égale à 0,10% masse.

On notera que la présente invention est utilisable pour les différentes versions de la norme ASTM D7157 existantes ou à venir, la méthode d'estimation du pouvoir solvant So pouvant être mise en œuvre dès lors que ce pouvoir solvant tel que défini dans cette norme (et notamment dans la version ASTM D7157-18 (révision 2018)) n'est pas mesurable selon l'une des versions de la norme. Bien entendu, on utilisera une même version de la norme ASTM D7157 pour établir la corrélation de la méthode d'estimation et pour la mise en œuvre des étapes de la méthode de prédiction selon l'invention.

Description détaillée de l'invention

La méthode selon l'invention permet de prédire la stabilité d'un mélange comprenant au moins un flux hydrocarboné contenant des asphaltènes et au moins un fluxant dont le pouvoir solvant So n'est pas mesurable selon la norme ASTM D7157, notamment selon la norme ASTM D7157-18 (Révision 2018) ou selon d'autres versions de la norme. L'invention est particulièrement avantageuse pour prédire la stabilité de mélanges tels que les combustibles lourds, notamment pour la marine.

A cet effet, le pouvoir solvant So du ou des fluxants est estimé au moyen de la méthode d'estimation selon l'invention à partir de caractéristiques physiques des fluxants.

La teneur en asphaltènes des flux hydrocarbonés ou fluxants peut être déterminée en utilisant la norme ASTM D6560 (janvier 2017).

Etape (a) d'établissement de la corrélation

L'étape (a) consiste à établir une corrélation exprimant le pouvoir solvant So d'un fluxant ou d'un mélange de fluxants en fonction de la viscosité cinématique à 50°C, la viscosité cinématique à 100°C et la masse volumique à 15°C dudit fluxant ou mélange de fluxants.

Cette étape (a) prévoit au cours d'une sous étape (i) de mesurer la viscosité cinématique à 50°C, la viscosité cinématique à 100°C et la masse volumique à 15°C d'une pluralité de fluxants et/ou de mélanges de fluxants.

Ces fluxants seront avantageusement choisis parmi les types de fluxants utilisables pour une application particulière.

Par exemple, notamment pour réaliser des mélanges de type combustible lourd, en particulier d'application marine, les fluxants peuvent être choisis parmi :

- VGO (Vacuum gasoil): coupe lourde vaporisable issue de la distillation sous vide d'un résidu atmosphérique. L'intervalle de températures d'ébullition de cette coupe est habituellement de 360-380 à 540-600°C,
- HCO (Heavy cycle oil) : coupe lourde (distillat lourd) issue d'une unité de craquage catalytique (FCC, Fluid Catalytic cracking),
- LCO (Light cycle oil) : coupe de type gazole (distillat moyen) issue d'une unité de craquage catalytique (FCC),
- HCCS (Heavy Catalytic Cracked Spirit) et HCN (Heavy Cracked Naphtha): essence lourde (Heavy naphta) correspondant habituellement à la coupe 160-220°C d'une unité FCC,
- Bleed : fraction lourde (très paraffinique) issue de l'hydrocraquage d'une charge de type distillat, correspondant habituellement à un fond d'unité,
- Kérosène : coupe issue de la distillation atmosphérique, présentant habituellement un point initial de distillation de 150 à 180°C et un point final de distillation de 225 à 250°C,
- Gazole (gasoil en anglais) : coupe distillat issue de la distillation atmosphérique, sous-vide ou encore du viscoréducteur, présentant habituellement un point initial de distillation de 220°C à 240°C et un point final de distillation de 350 à 380°C,
- et les mélanges d'un ou plusieurs des produits listés ci-dessus.

A titre d'exemple, les fluxants peuvent être des flux hydrocarbonés de masses volumiques à 15°C de 833,0 à 995,0 kg/m$^3$ et de viscosités de 0,8810 à 104,7mm$^2$/s à 50°C et de 0,5490 à 13,10mm$^2$/s à 100°C.

Cette sous étape (i) peut être mise en œuvre sur une pluralité de fluxants, de mélanges de fluxants ou les deux. Les mélanges de fluxants utilisés peuvent être des mélanges de deux ou trois fluxants, bien que des mélanges à plus de trois fluxants puissent être envisagés.

On réalise alors les mesures de viscosité cinématique à 50°C et 100°C et de masse volumique à 15°C de ces fluxants et/ou mélanges de fluxants.

L'étape (a) comprend également une étape (ii) dans laquelle on prévoit des flux hydrocarbonés dont il est possible de mesurer une valeur de pouvoir solvant So selon la norme ASTM D7157, notamment selon la norme ASTM D7157-18 (Révision 2018) ou selon une autre version de la norme. En général, les autres paramètres S-value et Sa de ces flux hydrocarbonés sont également mesurables selon la norme ASTM D7157, notamment selon la norme ASTM D7157-18 (Révision 2018) ou selon une autre version.

Les flux hydrocarbonés seront avantageusement choisis parmi les flux hydrocarbonés utilisables pour une application combustible particulière.

Avantageusement, le ou les flux hydrocarbonés peuvent être choisis parmi :

- les résidus atmosphériques ou des résidus sous vide issus de la distillation du pétrole brut,
- les effluents, notamment les résidus, issus des procédés de conversion thermique, tel que le procédé de viscoréduction,
- les effluents, notamment les résidus ou la coupe slurry, issus de procédés de craquage catalytique, tel que le procédé FCC (« Fluid Catalytic Cracking » ou craquage catalytique fluide,
- les effluents, notamment les résidus, issus des procédés d'hydrotraitement, d'hydrocraquage, d'hydroconversion profonde, du procédé ARDS, du procédé VRDS,
- les brais issus de procédés de séparation physique, tel que le désasphaltage,
- les mélanges de deux ou plus des produits pétroliers listés ci-dessus.

Les sous étapes (i) et (ii) peuvent indifféremment être mises en œuvre simultanément ou l'une après l'autre dans n'importe quel ordre.

On met ensuite en œuvre la sous étape (iii) au cours de laquelle on prépare au moins deux mélanges de proportions différentes de chacun des flux hydrocarbonés avec chacun des fluxants et/ou mélanges de fluxants.

Les mélanges pouvant être préparés sont ainsi :

- au moins deux mélanges en différentes proportions d'un flux hydrocarboné et d'un fluxant,
- au moins deux mélanges en différentes proportions de deux ou plus flux hydrocarbonés et d'un fluxant,
- au moins deux mélanges en différentes proportions de deux ou plus flux hydrocarbonés et de deux ou plus fluxants,
- au moins deux mélanges en différentes proportions d'un flux hydrocarboné et de deux ou plus fluxants.

De préférence, on n'utilisera pas plus de deux ou trois flux hydrocarbonés et/ou deux ou trois fluxants dans les mélanges, bien que des mélanges plus complexes puissent être envisagés.

Ces mélanges étant préparés, on mesure au cours de la sous étape (iv), pour chacun des mélanges comprenant un même fluxant ou mélange de fluxants, la valeur du pouvoir So, notamment selon la norme ASTM D7157.

Puis on trace, pour chaque flux hydrocarboné (ou mélange de flux hydrocarbonés), une droite représentant cette valeur de So en fonction de la proportion de fluxant ou de mélange de fluxants dans le mélange. Autrement dit, l'axe des abscisses représente la proportion (pourcentage ou fraction) de fluxant (de 0 à 100% - généralement % massique) et l'axe des ordonnées représente la valeur du pouvoir solvant So. On pourra choisir des mélanges comprenant des proportions de fluxant ou de mélange de fluxants telles que la valeur So du mélange est mesurable selon la norme ASTM D7157. Ainsi, de préférence, la proportion du mélange en flux hydrocarboné n'est pas nulle, par exemple supérieure à 10%, 20%, 30% ou 40% massique.

Pour chaque droite tracée, on détermine alors une valeur du pouvoir solvant So du fluxant ou du mélange de fluxants par extrapolation à une proportion de fluxant (ou mélange de fluxants) de 100%. On notera que la préparation de plus de deux mélanges en proportions différentes pour chaque fluxant/mélange de fluxants peut permettre d'améliorer la précision de la valeur du pouvoir solvant So obtenue par extrapolation. Pour une meilleure précision, il peut également être préférable de choisir des mélanges dont les proportions sont relativement distinctes, par exemple des proportions différant d'au moins 10%, 20% ou 30%.

Enfin, on calcule une valeur moyenne de ces valeurs de So pour chaque fluxant et/ou mélange de fluxants.

Les différentes données mesurées ou déterminées pour chaque fluxant et/ou mélange de fluxants, à savoir les valeurs moyennes du pouvoir solvant So, les valeurs de viscosité cinématique à 50°C, de viscosité cinématique à 100°C et de masse volumique à 15°C sont ensuite traitées par un traitement statistique tel que prévu dans la sous étape (v) de la méthode de prédiction selon l'invention afin d'établir la corrélation définie plus haut.

Pour une meilleure précision de la corrélation, il est préférable d'effectuer le traitement statistique sur un nombre statistiquement significatif de fluxants et/ou de mélanges de fluxants, autrement dit un nombre suffisant pour que le résultat soit représentatif. A titre d'exemple, le nombre de fluxants et/ou de mélanges de fluxants peut être d'au moins 20 ou 30 ou 40 ou 50 ou 60 ou 70 ou 80 ou 90 ou 100, de préférence d'au moins 100.

Ce traitement statistique peut notamment être un traitement de régression linéaire multiple.

A titre d'exemple, cette corrélation peut se présenter sous la forme :

$$So = A + B.v_{50} + C.v_{100} + D.CCAI \qquad \text{(Equation 4)}$$

où :

$A$, $B$, $C$, $D$ : coefficients déterminés par le traitement statistique,
$v_{50}$ : Viscosité cinématique (en mm$^2$/s) à 50°C,
$v_{100}$ : Viscosité cinématique (en mm$^2$/s) à 100°C,
$CCAI$: "Calculated Carbon Aromaticity Index" (indice calculé d'aromaticité du carbone), défini par:

$$CCAI = \rho_{15} - 81 - 141.Log[Log(v_{50} + 0,85)] - 483.Log\,\frac{T+273}{323} \qquad \text{(Equation 5)}$$

où

$\rho_{15}$ : masse volumique à 15°C (en kg/m$^3$),
$T$ : température (en °C).

Une autre corrélation utilisable est :

$$So = A' + B'.v_{50} + C'.v_{100} + D'.\rho_{15} + E'.Log[Log(v_{50} + 0,85)] \qquad \text{(Equation 6)}$$

où :
$A'$, $B'$, $C'$, $D'$, $E'$: coefficients déterminés par le traitement statistique, les autres paramètres étant tels que définis plus haut.

Etape (b) d'utilisation de la corrélation

Cette étape (b) utilise la corrélation pour estimer la valeur du pouvoir solvant So d'un fluxant ou d'un mélange de fluxants dont on a mesuré la viscosité cinématique à 50°C, la viscosité cinématique à 100°C et la masse volumique à 15°C.

Il suffit ainsi de connaître les valeurs de viscosité cinématique à 50°C, la viscosité cinématique à 100°C et la masse volumique à 15°C d'un fluxant/mélange de fluxants pour estimer la valeur du pouvoir solvant So de ce dernier.

On notera que les valeurs de masse volumique à 15°C, de viscosité cinématique à 50°C et de viscosité cinématique à 100°C mesurées des fluxants/mélange de fluxants choisis pour établir la corrélation au cours de l'étape (a)(i) sont chacune comprises dans des intervalles respectivement de masse volumique, de viscosité cinématique à 50°C et de viscosité cinématique à 100°C dont les bornes correspondent aux valeurs minimales et maximales mesurées sur l'ensemble de ces fluxants/mélanges de fluxants. Cette étape b) peut préférentiellement être mise en œuvre pour estimer la valeur du pouvoir solvant So d'un fluxant ou d'un mélange de fluxants dont la masse volumique à 15°C, la viscosité cinématique à 50°C et la viscosité cinématique à 100°C sont chacune comprises dans les intervalles correspondants définis ci-dessus ou sont proches de ces intervalles. Par « valeur proche d'un intervalle », on entend une valeur supérieure/inférieure aux bornes maximale/minimale de cet intervalle de 1 à 10%, de 1 à 5% ou de 5 à 10%.

Méthode de prédiction de la stabilité d'un mélange

Il est dès lors possible de prédire la stabilité d'un mélange en calculant la valeur du pouvoir solvant $So_{mélange}$ d'un mélange et la valeur de la peptisabilité des asphaltènes $Sa_{mélange}$ d'un mélange puis en calculant la valeur de la S-value du mélange $S_{mélange}$ en appliquant les lois de mélanges (équations (1) ou (1bis) et (2)) et la définition de la S-value (équation (3)), ceci même lorsque le mélange contient des constituants dont on ne peut mesurer les paramètres de stabilité selon la norme ASTM D7157, notamment selon la norme ASTM D7157-18 (Révision 2018) ou selon une autre version. Il suffit en effet de mettre en œuvre la méthode d'estimation selon l'invention précédemment décrite.

On peut alors prédire qu'un mélange sera stable si la valeur $S_{mélange}$ est supérieure ou égale à une valeur cible et instable si la valeur $S_{mélange}$ est inférieure à la même valeur cible.

Cette valeur cible est égale à la valeur 1,5 augmentée ou réduite d'une quantité prédéterminée nulle ou non nulle.

Cette quantité prédéterminée peut être nulle ou correspondre à un pourcentage de la valeur cible.

Cette quantité prédéterminée est par exemple une quantité correspondant à 0 à 50% ou 1 à 50% ou 5 à 40% ou 5 à 30% ou 5 à 20% ou 5 à 15% de la valeur cible ou à un pourcentage de la valeur cible compris dans tout intervalle défini par une ou plusieurs des bornes précitées.

Dans un mode de réalisation particulier, on peut prédire que le mélange est instable si la valeur $S_{mélange}$ est inférieure à une valeur seuil égale à la valeur cible réduite d'une quantité correspondant à une marge d'erreur. Ceci revient à augmenter la réserve de stabilité et réduire davantage les risques de mauvaise prédiction. Cette marge d'erreur, non nulle, peut correspondre à un pourcentage de la valeur cible.

A titre d'exemple, cette marge d'erreur peut représenter 1 à 50% ou 5 à 40% ou 5 à 30% ou 5 à 20% ou 5 à 15% de la valeur cible ou un pourcentage de la valeur cible compris dans tout intervalle défini par une ou plusieurs des bornes précitées.

Dans ce cas, lorsque la valeur $S_{mélange}$ est supérieure ou égale à la valeur seuil et inférieure à la valeur cible, on vérifie que le mélange est stable si sa teneur en sédiments potentiels mesurée selon la norme ISO 10307-2A :2009 est inférieure ou égale à 0,10% masse.

Quel que soit le mode de réalisation, de préférence, la valeur cible peut être égale à 1,5. La méthode de prédiction selon l'invention peut être utilisée pour un mélange contenant un ou plusieurs flux hydrocarbonés contenant des asphaltènes, notamment du type précédemment décrit en référence à la méthode d'estimation du pouvoir solvant, et un ou plusieurs fluxants, notamment du type précédemment décrit en référence à la méthode d'estimation du pouvoir solvant.

On notera que parmi les deux ou plus flux hydrocarbonés contenant des asphaltènes, l'un des flux hydrocarbonés peut jouer le rôle d'un fluxant vis-à-vis du ou des autres flux hydrocarbonés.

On peut ainsi prédire la stabilité d'un mélange contenant à la fois un ou plusieurs fluxants (de pouvoir solvant So mesurable selon la norme ASTM D7157, notamment selon la norme ASTM D7157-18 (Révision 2018) ou selon une autre version) et un ou plusieurs fluxants de pouvoir solvant So non mesurable selon la norme ASTM D7157, notamment selon la norme ASTM D7157-18 (Révision 2018).

Exemple : calcul de la S-value d'un mélange

On établit une corrélation de la forme :

$$So = A' + B'.v_{50} + C'.v_{100} + D'.\rho_{15} + E'.Log[Log(v_{50} + 0,85)] \text{ (Equation 6)}$$

au moyen d'un traitement statistique par régression linéaire multiple, tel que précédemment décrit.

La corrélation a été développée à partir de fluxants de masses volumiques à 15°C comprises de 833,0 à 995,0 kg/m$^3$ et de viscosités cinématiques comprises de 0,8810 à 104,7mm$^2$/s à 50°C et de 0,5490 à 13,10mm$^2$/s à 100°C en utilisant les normes suivantes :

ASTM D7157-18 (Révision 2018),
NF EN ISO 3104 (janvier 2018),
NF EN ISO 12185 (décembre 1996).

Cette corrélation est ensuite utilisée pour déterminer le pouvoir solvant de deux fluxants de pouvoir solvant So non mesurable selon la norme ASTM D7157-18 (Révision 2018) appelés F1 et F2. Ces fluxants présentent une masse volumique à 15°C comprise dans l'intervalle de 833,0 et 995,0 kg/m$^3$.

On peut alors prédire la S-value d'un mélange de ces fluxants F1 et F2 avec des flux hydrocarbonés C1 et C2 contenant ici plus de 0,5%m d'asphaltènes à partir des lois de mélange au moyen des équations (1) à (3).

Le tableau 1 ci-dessous rassemble les valeurs S, Sa et So mesurées pour les constituants C1 et C2 selon la norme ASTM D7157-18 (Révision 2018), les valeurs de So estimées pour les fluxants F1 et F2 au moyen de la corrélation linéaire précitée correspondant à l'équation (6) et établie selon l'invention et les valeurs S, Sa et So calculées pour un mélange des constituants C1, C2, F1, F2 dans les proportions indiquées dans le tableau.

On notera que la S-value du mélange est supérieure à 1,5, ce qui permet de prédire que le mélange est stable en considérant que la valeur cible est égale à 1,5.

Tableau 1

| | S-Value | | | % asph (%m) | % masse |
|---|---|---|---|---|---|
| | S | Sa | So | | |
| C1 | 3,28 | 0,8 | 0,65 | 0,9453 | 60,8 |
| C2 | 1,58 | 0,24 | 1,2 | 0,8377 | 17,0 |
| F1 | | | -0,11 | 0 | 9,2 |
| F2 | | | 0,33 | 0 | 13,0 |
| Mélange | 2,03 | 0,69 | 0,63 | 0,717 | 100,0 |

Mesure des asphaltènes selon la norme ASTM D6560 (janvier 2017).

**Revendications**

1.  Méthode d'estimation de la valeur du pouvoir solvant So d'un fluxant ou d'un mélange de fluxants pour composition combustible, la méthode comprenant :

    (a) établir une corrélation exprimant le pouvoir solvant So d'un fluxant ou d'un mélange de fluxants en fonction de la viscosité cinématique à 50°C, la viscosité cinématique à 100°C et la masse volumique à 15°C dudit fluxant ou mélange de fluxants,
    (b) utiliser ladite corrélation pour estimer la valeur du pouvoir solvant So d'un fluxant ou d'un mélange de fluxants dont on a mesuré la viscosité cinématique à 50°C, la viscosité cinématique à 100°C et la masse volumique à 15°C,

    l'étape (a) d'établissement de la corrélation comprenant :

    (i) mesurer la viscosité cinématique à 50°C, la viscosité cinématique à 100°C et la masse volumique à 15°C d'une pluralité de fluxants et/ou de mélanges de fluxants,
    (ii) prévoir des flux hydrocarbonés dont il est possible de mesurer une valeur de pouvoir solvant So selon la norme ASTM D7157,
    (iii) préparer au moins deux mélanges de différentes proportions de chacun des flux hydrocarbonés avec chacun des fluxants et mélanges de fluxants,
    (iv) pour chacun des mélanges comprenant un même fluxant ou mélange de fluxants, mesurer la valeur du pouvoir solvant So et tracer, pour chaque flux hydrocarboné, une droite représentant cette valeur du pouvoir solvant So en fonction de la proportion de fluxant ou de mélange de fluxants dans le mélange et, pour chaque droite tracée, déterminer une valeur du pouvoir solvant So du fluxant ou du mélange de fluxants par extrapolation à une proportion de fluxant de 100%, puis calculer une valeur moyenne de ces valeurs de pouvoir solvant So pour chaque fluxant et mélange de fluxants,
    (v) établir ladite corrélation par un traitement statistique des valeurs moyennes de pouvoir solvant So, des valeurs de viscosité cinématique à 50°C, de viscosité cinématique à 100°C et de masse volumique à 15°C de tous les fluxants et mélanges de fluxants.

2.  Méthode d'estimation selon la revendication 1, dans laquelle le traitement statistique mis en œuvre à l'étape (v) est un traitement de régression linéaire multiple.

3.  Méthode d'estimation selon la revendication 1 ou 2, dans laquelle les mélanges de fluxant sont des mélanges de deux ou trois fluxants.

4.  Méthode d'estimation selon l'une quelconque des revendications 1 à 3, dans laquelle les flux hydrocarbonés sont choisis parmi

    - les résidus atmosphériques ou des résidus sous vide issus de la distillation du pétrole brut,
    - les effluents, notamment les résidus, issus des procédés de conversion thermique, tel que le procédé de viscoréduction,

- les effluents, notamment les résidus ou la coupe slurry, issus de procédés de craquage catalytique, tel que le procédé de craquage catalytique fluide,
- les effluents, notamment les résidus, issus des procédés d'hydrotraitement, d'hydrocraquage, d'hydroconversion profonde, du procédé ARDS, du procédé VRDS,
- les brais issus de procédés de séparation physique, tel que le désasphaltage,
- les mélanges de deux ou plus des produits pétroliers listés ci-dessus.

**5.** Méthode d'estimation selon l'une quelconque des revendications 1 à 4, dans laquelle les fluxants sont des flux hydrocarbonés.

**6.** Méthode d'estimation selon l'une quelconque des revendications 1 à 5, dans laquelle les fluxants sont choisis parmi : VGO, HCO, LCO, HCCS, HCN, bleed d'hydrocraquage d'une charge de type distillat, kérosène, gazole et les mélanges d'un ou plusieurs de ces produits.

**7.** Méthode d'estimation selon l'une quelconque des revendications 1 à 6, dans laquelle les valeurs de masse volumique à 15°C, de viscosité cinématique à 50°C et de viscosité cinématique à 100°C mesurées des fluxants choisis pour établir la corrélation sont chacune comprises dans des intervalles respectivement de masse volumique, de viscosité cinématique à 50°C et de viscosité cinématique à 100°C dont les bornes correspondent aux valeurs minimale et maximale mesurées, et l'étape b) est mise en œuvre pour estimer la valeur du pouvoir solvant So d'un fluxant ou d'un mélange de fluxants dont la masse volumique à 15°C, la viscosité cinématique à 50°C et la viscosité cinématique à 100°C sont comprises dans l'intervalle correspondant ou proches de cette intervalle.

**8.** Méthode de prédiction de la stabilité d'un mélange comprenant au moins un flux hydrocarboné contenant des asphaltènes et au moins un fluxant, comprenant :

- estimer la valeur du pouvoir solvant So dudit au moins un fluxant en mettant en œuvre la méthode selon l'une quelconque des revendications 1 à 7,
- mesurer la valeur du pouvoir solvant So et la valeur de la peptisabilité des asphaltènes Sa dudit au moins un flux hydrocarboné selon la norme ASTM D7157, en utilisant la même version de la norme que celle mise en œuvre dans la méthode d'estimation selon l'une quelconque des revendications 1 à 7,
- calculer la valeur du pouvoir solvant $So_{mélange}$ du mélange et la valeur la valeur de la peptisabilité des asphaltènes $Sa_{mélange}$ du mélange en utilisant les lois de mélange suivantes :

$$So_{mélange} = \sum x_i \times So_i \qquad \text{(équation 1)}$$

ou

$$\text{ou } So_{mélange} = \frac{\sum x_i \times (100 - ASP_i) \times So_i}{\sum x_i \times (100 - ASP_i)} \text{ (équation 1bis),}$$

et

$$Sa_{mélange} = \frac{\sum x_i \times ASP_i \times Sa_i}{\sum x_i \times ASP_i} \text{ (équation 2)}$$

où :

$x_i$ est la fraction massique du composant i,
$ASP_i$ est la teneur en asphaltènes (%m) du composant i,
$So_i$ est le pouvoir solvant du composant i,
$Sa_i$ est la peptisabilité du composant i,

- calculer la valeur de la S-value du mélange $S_{mélange}$ définie comme étant égale à

$$S_{mélange} = \frac{So_{mélange}}{1-Sa_{mélange}} \text{ (équation 3),}$$

(équation 3),

- prédire que :

le mélange est stable si la valeur $S_{mélange}$ est supérieure ou égale à une valeur cible,
le mélange est instable si la valeur $S_{mélange}$ est inférieure à la même valeur cible,
cette valeur cible étant égale à la valeur 1,5 augmentée ou réduite d'une quantité prédéterminée nulle ou non nulle.

9. Méthode de prédiction de la stabilité d'un mélange selon la revendication 8, dans lequel on prédit que le mélange est instable si la valeur $S_{mélange}$ est inférieure à une valeur seuil égale à la valeur cible réduite d'une quantité correspondant à une marge d'erreur.

10. Méthode de prédiction de la stabilité d'un mélange selon la revendication 9, dans lequel, lorsque la valeur $S_{mélange}$ est supérieure ou égale à la valeur seuil et inférieure à la valeur cible, on vérifie que le mélange est stable si sa teneur en sédiments potentiels mesurée selon la norme ISO 10307-2A :2009 est inférieure ou égale à 0,10% masse.

Europäisches
Patentamt

European
Patent Office

Office européen
des brevets

# RAPPORT DE RECHERCHE EUROPEENNE

Numéro de la demande

EP 19 30 6674

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 2017/058216 A1 (BALASHANMUGAM SOBAN [GB] ET AL) 2 mars 2017 (2017-03-02) * exemples 1-4 * ----- | 1-10 | INV. G01N33/28 G01N11/00 |
| A | Marzie Derakhshesh: "Asphaltene Aggregation and Fouling Behavior", A thesis submitted to the Faculty of Graduate Studies and Research in partial fulfillment of the requirements for the degree of Doctor of Philosophy in Chemical Engineering Department of Chemical and Materials Engineering, University of Alberta, 1 janvier 2012 (2012-01-01), pages 1-194, XP055332146, Extrait de l'Internet: URL:https://era.library.ualberta.ca/files/cqr46r0958/Derakhshesh_Marzie_Fall 2012.pdf [extrait le 2017-01-04] * alinéa [3.3.1]; figure 3.2 * ----- | 1-10 | |
| A | US 2018/156772 A1 (BALASHANMUGAM SOBAN [GB] ET AL) 7 juin 2018 (2018-06-07) * example 1 * ----- | 1-10 | DOMAINES TECHNIQUES RECHERCHES (IPC) G01N |
| A | US 2017/261446 A1 (ADAM-BERRET MATTHIEU [BE] ET AL) 14 septembre 2017 (2017-09-14) * revendication 1 * ----- | 1-10 | |
| A | US 5 475 612 A (ESPINOSA ALAIN [FR] ET AL) 12 décembre 1995 (1995-12-12) * exemples 10 et 11 * ----- | 1-10 | |

Le présent rapport a été établi pour toutes les revendications

| Lieu de la recherche | Date d'achèvement de la recherche | Examinateur |
|---|---|---|
| La Haye | 10 juillet 2020 | Martin, Hazel |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
.......................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 19 30 6674

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-07-2020

| Document brevet cité au rapport de recherche | | Date de publication | Membre(s) de la famille de brevet(s) | | Date de publication |
|---|---|---|---|---|---|
| US 2017058216 | A1 | 02-03-2017 | CA | 2996950 A1 | 09-03-2017 |
| | | | CN | 107923901 A | 17-04-2018 |
| | | | EP | 3344990 A1 | 11-07-2018 |
| | | | RU | 2018111103 A | 07-10-2019 |
| | | | US | 2017058216 A1 | 02-03-2017 |
| | | | WO | 2017040042 A1 | 09-03-2017 |
| | | | ZA | 201801295 B | 30-01-2019 |
| US 2018156772 | A1 | 07-06-2018 | CA | 3044527 A1 | 07-06-2018 |
| | | | CN | 110036294 A | 19-07-2019 |
| | | | EP | 3548886 A1 | 09-10-2019 |
| | | | US | 2018156772 A1 | 07-06-2018 |
| | | | WO | 2018102301 A1 | 07-06-2018 |
| | | | ZA | 201903458 B | 26-02-2020 |
| US 2017261446 | A1 | 14-09-2017 | CA | 2957249 A1 | 18-02-2016 |
| | | | EP | 3180630 A1 | 21-06-2017 |
| | | | FR | 3024902 A1 | 19-02-2016 |
| | | | JP | 2017530340 A | 12-10-2017 |
| | | | US | 2017261446 A1 | 14-09-2017 |
| | | | WO | 2016023984 A1 | 18-02-2016 |
| US 5475612 | A | 12-12-1995 | AU | 603920 B2 | 29-11-1990 |
| | | | CA | 1325732 C | 04-01-1994 |
| | | | DE | 3882847 D1 | 09-09-1993 |
| | | | DE | 3882847 T2 | 18-11-1993 |
| | | | EP | 0305090 A2 | 01-03-1989 |
| | | | ES | 2041801 T3 | 01-12-1993 |
| | | | JP | 2880171 B2 | 05-04-1999 |
| | | | JP | H01113636 A | 02-05-1989 |
| | | | NO | 300027 B1 | 17-03-1997 |
| | | | US | 5475612 A | 12-12-1995 |

EPO FORM P0460

Pour tout renseignement concernant cette annexe : voir Journal Officiel de l'Office européen des brevets, No.12/82